# EUROPÄISCHE PATENTSCHRIFT

(11) **EP 1 905 469 B1**
(45) Veröffentlichungstag und Bekanntmachung des Hinweises auf die Patenterteilung: **22.05.2013**
(21) Anmeldenummer: 07018051.8
(22) Anmeldetag: 14.09.2007
(51) Int. Cl.: A61M 15/00, B05B 17/06

(54) **Mikrodosiervorrichtung für ein flüssiges Medium**
Micro dosing device for a liquid medium
Dispositif de microdosage pour un milieu liquide

(30) Priorität: 29.09.2006 DE 102006047658
(43) Veröffentlichungstag der Anmeldung: 02.04.2008
(73) Patentinhaber: Aptar Radolfzell GmbH, 78315 Radolfzell (DE)
(72) Erfinder: Helmlinger, Michael, 78315 Radolfzell (DE)
(74) Vertreter: Patentanwälte Ruff, Wilhelm, Beier, Dauster & Partner

(56) Entgegenhaltungen:
- EP-A- 0 739 654
- EP-A- 1 502 606
- WO-A-97/07896
- GB-A- 1 575 887
- US-A1- 2005 207 917

## Beschreibung

Die Erfindung betrifft eine Mikrodosiervorrichtung für ein flüssiges Medium mit einem Dosierraum sowie mit einer elektrisch oder elektronisch aktivierbaren Vibrationseinheit, die wenigstens eine Begrenzungsfläche des Dosierraumes für einen Austragvorgang eines Mediumvolumens in Schwingungen versetzen kann, sowie mit einem Mediumspeicher, der mittels wenigstens eines Strömungskanales mit dem Dosierraum verbunden ist.

Eine derartige Mikrodosiervorrichtung ist aus der DE 10 2004 006 452 A1 bekannt. Die bekannte Mikrodosiervorrichtung saugt durch entsprechende Druckdifferenzen und durch Kapillarwirkung das auszutragende Mediumvolumen aus einer als Kapillare ausgeführten Vorratskammer in den Dosierraum. Zudem ist eine Befüllungseinrichtung vorgesehen, die Flüssigkeit in die Vorratskammer und in die Dosierkammer fördert, um die Vorratskammer und die Dosierkammer zu befüllen.

Aufgabe der Erfindung ist es, eine Mikrodosiervorrichtung der eingangs genannten Art zu schaffen, die einfach handhabbar und zuverlässig dosierbar ist.

Diese Aufgabe wird dadurch gelöst, dass dem wenigstens einen Strömungskanal eine manuell betätigbare Fördereinrichtung zum Zuführen von Medium in den Dosierraum und/oder zum Rückführen von Medium aus dem Dosierraum in den Mediumspeicher zugeordnet ist. Durch die erfindungsgemäße Lösung ist es für eine Bedienperson möglich, bei Bedarf den Dosierraum zu befüllen, um anschließend bei vollständig befülltem Dosierraum eine Applikation starten zu können. Dadurch ist eine zuverlässige und gleichbleibende Dosierung gewährleistet. Dies ist insbesondere bei pharmazeutischen und medizinisch wirksamen Medien vorteilhaft, um Über- oder Unterdosierungen bei einer entsprechenden Anwendung vermeiden zu können. Die Mediumzufuhr durch die Fördereinrichtung kann direkt oder indirekt erfolgen. Bei direkter Befüllung steht die Fördereinrichtung über einen Strömungskanal mit dem Dosierraum in Verbindung. Bei indirekter Befüllung dient die Fördereinrichtung dazu, indirekt - insbesondere durch Druckaufbau - auf das Medium derart einzuwirken, dass es von einem Mediumspeicher zu dem Dosierraum gefördert wird.

In Ausgestaltung der Erfindung ist eine Sensorik vorgesehen, die einen Befüllungsvorgang durch die Fördereinrichtung erfasst und entsprechende Signale an eine Steuereinheit weiterleitet, die die Vibrationseinheit in Abhängigkeit von den entsprechenden Signalen der Sensorik zu- oder abschaltet. Dadurch ist gewährleistet, dass ein Ausbringen des Mediums im Dosierraum erst dann erfolgt, wenn der Dosierraum vollständig befüllt ist. Sensiert wird nicht der Befüllungszustand des Dosierraumes, sondern vielmehr der Befüllungsvorgang durch die Fördereinrichtung. In vorteilhafter Weise umfasst die Sensorik wenigstens einen Wegsensor, der die Anfangs- und/oder Endpunkte eines Bewegungsvorganges der insbesondere mechanischen Fördereinrichtung erfasst. Falls die Befüllung durch die Fördereinrichtung indirekt über Druckbeaufschlagung erfolgt, wird die Druckbeaufschlagung sensiert. Statt eines Wegsensors kann insbesondere auch ein Drucksensor vorgesehen sein.

In weiterer Ausgestaltung der Erfindung umfasst die manuell betätigbare Fördereinrichtung eine mechanische Kolbenpumpe. Die Kolbenpumpe wird für einen entsprechenden Arbeitshub manuell durch eine Bedienperson betätigt. Ein Rückhub in die Ausgangslage erfolgt vorzugsweise durch Federkraft. Es ist aber auch möglich, bei der mechanischen Kolbenpumpe lediglich die Einleitung des Hubvorganges manuell zu starten und anschließend den Arbeitshub durch eine vorgespannte Federeinheit federbetätigt ablaufen zu lassen. Eine weitere Federeinheit kann dann für den Rückhub verantwortlich sein. Hierdurch wird benutzerunabhängig immer eine gleichbleibende Befüllung des Dosierraumes erreicht.

Falls dem Dosierraum zusätzlich eine insbesondere als Kapillare ausgebildete Vordosierkammer zugeordnet ist, ist das Fördervolumen der manuell betätigbaren, mechanischen Fördereinrichtung vorteilhaft so bemessen, dass mit einem einzigen Arbeitshub sowohl der Dosierraum als auch die Vordosierkammer befüllt werden können.

ln weiterer Ausgestaltung der Erfindung ist wenigstens ein Strömungskanal zwischen dem Dosierraum und dem Mediumspeicher und/oder zwischen dem Dosierraum und der Fördereinrichtung und/oder zwischen der Fördereinrichtung und dem Mediumspeicher mit wenigstens einer Ventilanordnung versehen. Dadurch ist gewährleistet, dass Fehlströmungen des Mediums vermieden werden. Hierdurch ist insbesondere eine Vermischung von im Bereich des Dosierraumes bereits mit Umgebungsluft in Kontakt geratenem, kontaminiertem Medium mit noch unkontaminiertem Medium aus dem Mediumspeicher vermeidbar.

In weiterer Ausgestaltung der Erfindung öffnet oder schließt die Ventilanordnung druckabhängig. In weiterer Ausgestaltung ist die Ventilanordnung als mechanisches Rückschlagventil ausgeführt. Dadurch wird eine ungewünschte Rückströmung oder Rücksaugung von Medium entgegen der gewünschten Strömungsrichtung vermieden.

In weiterer Ausgestaltung der Erfindung ist der Strömungskanal zwischen dem Mediumspeicher und dem Dosierraum mit einer Filtereinrichtung, insbesondere einer mikrobiologischen Filtereinrichtung, versehen. Dadurch ist gewährleistet, dass kein kontaminiertes Medium über den Strömungskanal in den Mediumspeicher eintreten kann. Das Medium im Mediumspeicher selbst, vorzugsweise eine entsprechende pharmazeutische Flüssigkeit, ist mikrobiologisch rein. Der Mediumspeicher ist derart dicht ausgeführt, dass keine Umgebungsgase in den Mediumspeicher eintreten können.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt schematisch eine Ausführungsform einer erfindungsgemäßen Mikrodosiervorrichtung, bei der eine direkte Befüllung eines Dosierraumes durch eine mechanische Fördereinrichtung erfolgt und
- Fig. 2: eine weitere Ausführungsform einer Mikrodosiervorrichtung: die nicht Teil der in Ansprüche 1-2 definierter Erfindung ist, bei der eine indirekte Befüllung des Dosierraumes über eine Druckbeaufschlagung eines Mediumspeichers erfolgt.

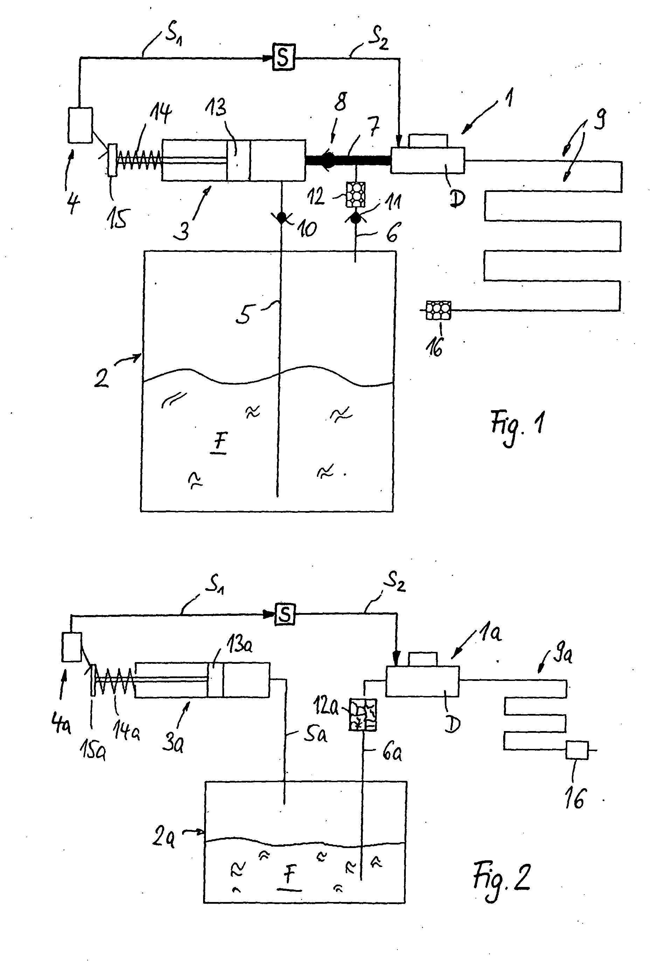

Eine Mikrodosiervorrichtung nach Fig. 1 dient dazu, flüssige Medien in geringen Mengen und hochgenau dosiert auszubringen. In besonders vorteilhafter Weise ist eine derartige Mikrodosiervorrichtung für medizinische Indikationen zum Ausbringen pharmazeutischer Flüssigkeiten vorgesehen. In gleicher Weise kann die Mikrodosiervorrichtung aber auch für andere Einsatzzwecke eingesetzt werden. Zur hochgenauen Dosierung weist die Mikrodosiervorrichtung einen Dosierraum D auf, der zu einer Seite hin über eine Vielzahl mikroskopisch kleiner Austragöffnungen zur Umgebung hin offen ist und an einer von der Austragseite abliegenden Seite mit einer Begrenzungsfläche versehen ist, der eine Vibrationseinheit zugeordnet ist. Die Vibrationseinheit ist vorzugsweise als Piezoelement ausgeführt und durch elektrische oder elektronische Steuermittel derart in Schwingungen versetzbar, dass die Begrenzungsfläche des Dosierraumes vibriert. Diese Vibrationen führen dazu, dass über entsprechende Kapillarkräfte das im Dosierraum befindliche, flüssige Medium durch die mikroskopisch kleinen Austragöffnungen hindurch zur Umgebung hin austritt. An den auch als Dosierkammer bezeichneten Dosierraum D ist eine Vordosierkammer 9 angeschlossen, die kommunizierend mit dem Dosierraum D in Verbindung steht und als Kapillare ausgeführt ist. Die Vordosierkammer 9 ist auf ihrer dem Dosierraum D abgewandten Endseite offen. Der offene Bereich ist mit einer mikrobiologischen Filtereinheit 16 versehen, um so einen Druckausgleich mit der Umgebung zu ermöglichen, gleichzeitig aber zu verhindern, dass kontaminierte Luft in die Vordosierkammer 9 gelangen kann.

Der grundsätzliche Aufbau der durch die Vibrationseinheit, den Dosierraum D, die Austragöffnungen und die Vordosierkammer gebildeten Austrageinheit entspricht der aus der DE 10 2004 006 452 A1 bekannten Baueinheit, so dass bezüglich weiterer Details ergänzend auf den Inhalt dieser Druckschrift verwiesen wird.

Um den Dosierraum D und die Vordosierkammer 9 befüllen zu können, ist der Dosierraum D auf einer der Vordosierkammer 9 abgewandten Seite mit einem Strömungskanal 7 verbunden, der über einen abgezweigten Rücklaufkanal 6, der ebenfalls einen Strömungskanal darstellt, an einen Mediumspeicher 2 angeschlossen ist. Der Strömungsquerschnitt des Strömungskanales 7 ist um ein Vielfaches größer als der Strömungsquerschnitt des Rücklaufkanales 6.

Der Mediumspeicher 2 speichert eine als Medium dienende Flüssigkeit F in hermetisch abgedichteten Zustand. In den Mediumspeicher 2 ragt ein als Ansaugkanal dienender Strömungskanal 5 hinein, dessen offener Ansaugstutzen unterhalb des Flüssigkeitsstandes der Flüssigkeit F angeordnet ist und somit permanent in die Flüssigkeit F eingetaucht ist, solange der Mediumspeicher 2 nicht im wesentlichen leer ist. Der Ansaugkanal 5 ist mit einer als Befüllungseinrichtung dienenden Fördereinrichtung 3 verbunden, die als manuell betätigbare Schubkolbenpumpe ausgebildet ist. Die Fördereinrichtung 3 weist einen Arbeitskolben 13 auf, der in einem entsprechenden Arbeitszylinder linear verschiebbar gelagert ist. Durch eine Verschiebung des Arbeitskolbens wird das Volumen eines Zylinderraumes des Arbeitszylinders in grundsätzlich bekannter Weise verändert. An den Zylinderraum des Arbeitszylinders schließt der Strömungskanal 7 an, der zu dem Dosierraum D führt. Zudem ist der Zylinderraum auch mit dem Ansaugkanal 5 verbunden, der in den Mediumspeicher 2 mündet. Eine Kolbenstange des Arbeitskolbens 13 ragt aus dem Arbeitszylinder hinaus und ist an ihrem außenliegenden Stirnende mit einem Anschlagblock 15 versehen, an dem sich eine Rückstellfederanordnung 14 abstützt. Die Rückstellfederanordnung 14 dient dazu, den Arbeitskolben 13 nach einem entsprechenden Funktionshub in eine Ausgangslage zurückzustellen.

Um eine Bewegung des Arbeitskolbens 13 aus seiner Ausgangslage gemäß Fig. 1 heraus oder in die Ausgangslage zurück zu erfassen, ist dem Anschlagblock 15 eine Sensorik 4, vorliegend in Form eines Endschalters, zugeordnet, der mittels einer Signalleitung S₁ an eine elektrische oder elektronische Steuereinheit S angeschlossen ist. Die Steuereinheit S steuert mittels einer Steuerleitung S₂ die Aktivierung oder Deaktivierung der Vibrationseinheit.

Dem Ansaugkanal 5 ist ein in Richtung des Zylinderraumes öffnendes Rückschlagventil 10 zugeordnet, das verhindert, dass bereits im Zylinderraum der Fördereinrichtung 3 befindliche Flüssigkeit in den Mediumspeicher 2 zurückfließen kann. Auch dem Strömungskanal 7 ist ein Rückschlagventil 8 zugeordnet, das zum Dosierraum D hin öffnet, um zu verhindern, dass Flüssigkeit aus dem Strömungskanal 7 und aus dem Dosierraum D zurück in den Zylinderraum der Fördereinrichtung 3 fließen kann. Schließlich ist auch der Rücklaufkanal 6 mit einem zum Mediumspeicher 2 hin öffnenden Rückschlagventil 11 versehen. Das Rückschlagventil 11 dient dazu, den Rücklaufkanal 6 zu schließen, sobald der Druck im Mediumspeicher 2 höher ist als im Strömungskanal 7. Dem Rückschlagventil 11 ist eine Filtereinrichtung 12 vorgeschaltet, die somit am Rücksaugkanal 6 zwischen Strömungskanal 7 und Rückschlagventil 11 angeordnet ist. Die Filtereinrichtung 12 stellt einen mikrobiologischen Filter dar. Dadurch wird verhindert, dass kontaminierte Flüssigkeit aus dem Dosierraum D und vom Strömungskanal 7 in den Mediumspeicher 2 zurückfließen kann. Die kontaminierenden Bestandteile bleiben in der Filtereinrichtung 12 hängen.

Durch die beschriebene Anordnung ist gewährleistet, dass die Flüssigkeit F im Mediumspeicher 2 rein und damit unkontaminiert bleibt.

Die Arbeitsweise der Mikrodosiervorrichtung nach Fig. 1 ist wie folgt:

Um den Dosierraum D und die Vordosierkammer 9 mit Flüssigkeit F zu füllen, wird zunächst über den Ansaugkanal 5 Flüssigkeit F in den Zylinderraum der Fördereinrichtung 3 angesaugt, indem der Arbeitskolben 13 einen entsprechenden Hub vollführt. Diese Erstbefüllung des Zylinderraumes kann als Priming-Vorgang bezeichnet werden. Es ist sinnvoll, dass trotz entsprechender Erfassung des entsprechenden Funktionshubes durch den Endschalter der Sensorik 4 während dieser Erstbefüllung kein Steuervorgang für die Vibrationseinheit erfolgt. Sobald der Zylinderraum befüllt ist, kann nun in einem nächsten Hub oder in mehreren Hüben der Dosierraum D und die Vordosierkammer 9 befüllt werden. Hierzu wird der Arbeitskolben 13 manuell in Richtung des Strömungskanales 7 gedrückt, wodurch der entsprechende Strömungsdruck das Rückschlagventil 8 öffnet und die Flüssigkeit in den Dosierraum D und die Vordosierkammer 9 strömt. Vorzugsweise ist ein Arbeitshub des Arbeitskolbens 13 und damit das Volumen des Zylinderraumes derart auf das Befüllungsvolumen des Dosierraumes D und der Vordosierkammer 9 abgestimmt, dass mit einem einzigen Hub des Arbeitskolbens 13 eine ausreichende Befüllung des Dosierraumes D und der Vordosierkammer 9 erfolgt. Durch den Druckaufbau innerhalb des Zylinderraumes wird zwangsläufig das Rückschlagventil 10 des Ansaugkanales 5 geschlossen, so dass keine Flüssigkeit in den Mediumspeicher 2 zurückgedrückt werden kann. Da die Strömungsquerschnitte des Strömungskanales 7 und des Rücklaufkanales 6 sich stark unterscheiden, wie oben ausgeführt wurde, ist zudem der Strömungswiderstand innerhalb des Rücklaufkanales 6 so groß, dass die Flüssigkeit in den Dosierraum D gedrückt wird, ohne einen Teilstrom in den Rücklaufkanal 6 abzuleiten.

Bei einem entsprechenden Rückhub des Arbeitskolbens 13 wird zwangsläufig bereits wieder Flüssigkeit F in den Zylinderraum gesaugt. Da das Rückschlagventil 8 des Strömungskanales 7 bei dem entsprechenden Rückhub, der nach Wegfall der manuellen Belastung durch die Rückstellkraft der Rückstellfederanordnung 14 erfolgt, geschlossen wird, kann keine Flüssigkeit mehr aus dem Dosierraum D oder aus dem Strömungskanal 7 zurückgesaugt werden. Nach den beschriebenen Hüben des Arbeitskolbens 13 sind nun der Dosierraum D und die Vordosierkammer 9 wie auch der Zylinderraum der Fördereinrichtung 3 befüllt. Sobald nun manuell ein erneuter Arbeitshub des Arbeitskolbens 13 eingeleitet wird, wird gleichzeitig mit der Einleitung der Kolbenbewegung über die Steuereinheit S die Vibrationseinheit aktiviert, so dass ein Austragvorgang erfolgt. Sobald der Anschlagblock 15 bei einem anschließenden Rückhub des Arbeitskolbens 13 wieder an dem Endschalter anschlägt, kann die Vibrationseinheit deaktiviert werden.

Es ist aber auch möglich, andere Ansteuerungsvorgänge der Vibrationseinheit durch die Steuereinheit S abhängig von entsprechenden Signalen der Sensorik vorzusehen. Falls die Steuereinheit S eine intelligente Elektronik umfasst, können hier entsprechend unterschiedliche Ansteuerprogramme einprogrammiert und ausgeführt werden. Insbesondere überflüssige Flüssigkeit im Strömungskanal 7 kann durch den Rücklaufkanal 6 in den Mediumspeicher 2 abfließen. Falls der Mediumspeicher 2 als geschlossener Druckbehälter gestaltet ist, entsteht bei Entnahme von Flüssigkeit F aus dem Mediumspeicher 2 zwangsläufig ein Unterdruck, der einen entsprechenden Ansaugdruck bewirken kann, um das Rückschlagventil 11 des Rücklaufkanales 6 zu öffnen und so überschüssige Flüssigkeit aus dem Strömungskanal 7 wie auch aus dem Dosierraum D und der Vordosierkammer 9 in den Mediumspeicher 2 zurückzusaugen. Dies ist insbesondere dann vorteilhaft, wenn bei einem entsprechenden Austragvorgang durch die Vibrationseinheit keine vollständige Entleerung des Dosierraumes D und/oder der Vordosierkammer erfolgt ist.

Vom grundsätzlichen Aufbau, wie z.B. aus der GB 1575887 A bekannt, her entspricht die Mikrodosiervorrichtung nach Fig. 2 der Mikrodosiervorrichtung nach Fig. 1, so dass ergänzend auf die Offenbarung der Ausführungsform nach Fig. 1 verwiesen wird. Funktionsgleiche Teile und Baueinheiten sind mit den gleichen Bezugszeichen wie bei der Ausführungsform nach Fig. 1, jedoch unter Hinzufügung des Buchstabens a versehen. Wesentlicher Unterschied bei der Ausführungsform nach Fig. 2 ist es, dass dort keine direkte Befüllung des Dosierraumes D und der Vordosierkammer 9a durch die Fördereinrichtung 3a erfolgt, sondern vielmehr eine indirekte Befüllung über entsprechende Druckbeaufschlagung des druckdichten Mediumspeichers 2a stattfindet. Hierzu ist ein Strömungskanal 6a vorgesehen, der den Mediumspeicher 2a mit dem Dosierraum D verbindet und als Ansaugkanal für die Flüssigkeit F gestaltet ist. Der Ansaugkanal 6a ragt analog dem Ansaugkanal 5 der Fig. 1 in die Flüssigkeit F hinein. Die Fördereinrichtung 3a hingegen ist über einen Druckkanal 5a lediglich nach Art eines Druckbehälters mit dem Mediumspeicher 2a verbunden, indem der entsprechende Druckkanal 5a lediglich in den oberen Bereich des Mediumspeichers 2a hineinragt, ohne zwangsläufig mit der Flüssigkeit F in Kontakt zu geraten.

Eine vorzugsweise manuelle Belastung des Kolbens 13a führt zu einer linearen Kolbenbewegung, die den Zylinderraum verkleinert und über den Druckkanal 5a den Mediumspeicher 2a unter Druck setzt.

Ein entsprechender Druckaufbau im Mediumspeicher 2a führt im Mediumspeicher zu einem Überdruck, der die Flüssigkeit F durch den Strömungskanal 6a in den Dosierraum D und in die Vordosierkammer 9a drückt. Eine vorzugsweise gleichzeitige Aktivierung der Vibrationseinheit zum Austragen der im Dosierraum D befindlichen Flüssigkeit bewirkt eine gewünschte Entleerung des Dosierraumes D und gegebenenfalls der Vordosierkammer 9a. Vorteilhaft sollten die Druckverhältnisse auch so gestaltet sein, dass kein permanentes Auslaufen von Flüssigkeit im Bereich der Austragöffnungen des Dosierraumes D erfolgt.

Während des Austragvorganges von Medium aus dem Dosierraum D zur Umgebung muss der Kolben 13a gegen die Rückstellkraft der Rückholfeder 14a gedrückt gehalten bleiben. In vorteilhafter Weise ist die Steuereinheit S so gestaltet, dass die Vibrationseinheit während der Druckbelastung durch den Kolben 13a aktiviert bleibt. Sobald die Belastung des Kolbens 13a entfernt wird, vorzugsweise durch Wegnahme der manuellen Belastung, stellt die Rückholfeder 14a den Kolben wieder in die unbelastete Ausgangslage gemäß Fig. 2 zurück. Dadurch entsteht aufgrund der miteinander kommunizierenden Räume, nämlich dem Zylinderraum und dem Raum im Mediumspeicher 2a, im Mediumspeicher 2a ein Unterdruck, der ein Rücksaugen von im Dosierraum D verbliebenen Mediumresten bewirkt. Um zu verhindern, dass Kontaminationen aus dem Dosierraum in den Mediumspeicher gelangen, ist eine Filtereinheit 12a vorgesehen, die vorzugsweise mikrobiologisch wirksam ist. Diese hält Kontaminationen zurück, so dass die Flüssigkeit im Mediumspeicher rein bleibt. Die Rückstellung des Kolbens 13a in die Ausgangslage bewirkt ein Sensorsignal der Sensoreinheit 4a, so dass die Vibrationseinheit über die Steuereinheit S abgeschaltet werden kann.

## Patentansprüche

1. Mikrodosiervorrichtung für ein flüssiges Medium mit einem Dosierraum (D) sowie mit einer elektrisch oder elektronisch aktivierbaren Vibrationseinheit (S), die wenigstens eine Begrenzungsfläche des Dosierraumes (D) für einen Austragvorgang eines Mediumvolumens in Schwingungen versetzen kann, sowie mit einem insbesondere druckdichten Mediumspeicher (2), der mittels wenigstens eines Strömungskanales mit dem Dosierraum (D) verbunden ist,
**dadurch gekennzeichnet, dass**
dem Strömungskanal (5, 7) eine manuell betätigbare Kolbenpumpe (3) zum Zuführen von Medium in den Dosierraum (D) zugeordnet ist, wobei in dem Strömungskanal (7) zwischen der Kolbenpumpe (3) und dem Dosierraum (D) ein mechanisches Rückschlagventil (8) vorgesehen ist, welches den Rückfluss von kontaminiertem Medium aus dem Dosierraum (D) in die Kolbenpumpe (3) verhindert, und wobei vom Strömungskanal (7), der zwischen Kolbenpumpe (3) und Dosierraum (D) verläuft, zwischen dem Rückschlagventil (8) und dem Dosierraum (D) ein weiterer Strömungskanal (6) zum Mediumspeicher (2) abzweigt, der mit einer Filtereinrichtung (12) versehen ist.

2. Mikrodosiervorrichtung nach Anspruch 1, **dadurch gekennzeichnet, dass** eine Sensorik (4) vorgesehen ist, die einen Befüllungsvorgang durch die Fördereinrichtung (3) erfasst und entsprechende Signale an eine Steuereinheit (S) weiterleitet, die die Vibrationseinheit in Abhängigkeit von entsprechenden Signalen der Sensorik zu- oder abschaltet.

## Claims

1. A microdosing device for a liquid medium with a dosing compartment (D) and with an electrically or electronically activatable vibration unit (S) which can cause at least one boundary area of the dosing compartment (D) to vibrate for a delivery procedure of a volume of medium and with a medium reservoir (2) which is, in particular, pressure-tight and which is connected to the dosing compartment (D) by means of at least one flow channel,
**characterized in that**
a manually operable piston pump (3) for conveying medium to the dosing compartment (D) is assigned to the flow channel (5, 7), wherein in the flow channel (7) is provided a mechanical non-return valve (8) between the piston pump (3) and the dosing compartment (D), to prevent backflow of contaminated medium from the dosing compartment (D) into the piston pump (3), and wherein from the flow channel (7), extending between the piston pump (3) and the dosing compartment (D), between the non-return valve (8) and the dosing compartment (D) another flow channel (6) branches off to the medium reservoir (2), and is provided with a filter device (12).

2. The microdosing device according to claim 1, **characterized in that** a sensor system (4) is provided which detects a filling operation by the conveyance device (3) and transmits associated signals to a control unit (S) which switches the vibration unit on or off in response to associated signals from the sensor system.

## Revendications

1. Dispositif de microdosage pour un médium liquide comprenant un espace de dosage (D) ainsi qu'une unité vibrante (S) activable électriquement ou électroniquement, laquelle peut faire entrer en oscillation au moins une surface de délimitation de l'espace de dosage (D) pour une opération d'extraction d'un volume de médium, et un accumulateur de médium (2) notamment hermétique à la pression qui est relié avec l'espace de dosage (D) au moyen d'au moins un canal d'écoulement,
**caractérisé en ce que**
au canal d'écoulement (5, 7) est associée une pompe à piston (3) à commande manuelle pour acheminer du médium dans la chambre de dosage (D), un clapet anti-retour mécanique (8) étant prévu dans le canal d'écoulement (7) entre la pompe à piston (3) et l'espace de dosage (D), lequel empêche le reflux du médium contaminé de l'espace de dosage (D) dans la pompe à piston (3), et, depuis le canal d'écoulement (7) qui s'étend entre la pompe à piston (3) et l'espace de dosage (D), entre le clapet anti-retour (8) et l'espace de dosage (D), un canal d'écoulement supplémentaire (6) bifurquant vers l'accumulateur de médium (2), lequel est muni d'un dispositif de filtrage (12).

2. Dispositif de microdosage selon la revendication 1, **caractérisé en ce qu'**il est prévu un dispositif de détection (4) qui capte une opération de remplissage par le dispositif de refoulement (3) et retransmet des signaux correspondants à une unité de commande (S), laquelle met en circuit ou hors circuit l'unité vibrante en fonction des signaux correspondants du dispositif de détection.
